# EUROPEAN PATENT APPLICATION

(11) **EP 3 777 801 A1**
(43) Date of publication of application: **17.02.2021**
(21) Application number: 19777680.0
(22) Date of filing: 28.03.2019
(51) Int. Cl.: A61F 13/515, A61F 13/511, A61F 13/512, A61F 13/514

(54) **ABSORBABLE ARTICLE**

(30) Priority: 28.03.2018 JP 2018061583
(71) Applicant: DAIO PAPER CORPORATION, Shikokuchuo-shi Ehime 799-0492 (JP)
(72) Inventor: NAGASHIMA, Mariko, Sakura-shi, Tochigi 329-1411 (JP)
(74) Representative: Schön, Christoph
(86) International application number: PCT/JP2019/013603
(87) International publication number: WO 2019/189574

(57) **Abstract**

Problem

To prevent a front-surface sheet from delaminating and tearing accompanied therewith when non-thermally fusible fiber is used in the front-surface sheet.

Solution

An absorber 4 is interposed between a front-surface sheet 3 and a back-surface sheet 2, and side sheets 7 are respectively disposed along a longitudinal direction on both side parts of a skin side. The front-surface sheet 3 contains non-thermally fusible fiber, and the back-surface sheet 2 and the side sheets 7 contain thermally fusible resin. Fusion parts 10 allowing the side sheets 7 and the back-surface sheet 2 to be thermally fused under a state of the front-surface sheet 3 interposed therebetween are arranged in areas corresponding to side parts outer than the absorber 4 in a width direction, and the areas include the front-surface sheet 3 interposed between the side sheets 7 and the back-surface sheet 2, and include at least front and back end parts of a sanitary napkin 1.

## Description

### Technical field

The present invention relates to an absorbable article such as a sanitary napkin, a panty liner, and an incontinence pad, and in particular relates to an absorbable article including a front-surface sheet containing non-thermally fusible fiber such as cotton fiber, allowing to prevent delamination from the front and back end parts thereof and tear accompanied therewith.

### Background technology

Conventionally, an absorbable article is known in which an absorber made of paper cotton such as crushed pulp is interposed between a liquid impermeable back-surface sheet such as a polyethylene sheet or a nonwoven fabric of laminated polyethylene sheet and a liquid permeable front-surface sheet.

Since the font-surface sheet forms a skin-contact surface, the front-surface sheet is required, for example, to be soft, to give dry feeling even after absorption of expelled body fluid, and to hardly irritate the skin. Although a synthetic fiber nonwoven fabric and a resin mesh sheet are widely used in the field of an absorbable article, a front-surface sheet made of synthetic fiber may cause itching, rash and other troubles. To solve such troubles, a front-surface sheet made of cotton fiber is proposed.

Patent Document 1 to be described below and others disclose an absorbent article including a front-surface sheet made of cotton fiber. Patent Document 1 to be described below discloses an absorbent article including a front-surface sheet containing non-thermally fusible fiber, a pair of side sheets disposed on a facing-skin surface of the front-surface sheet, so as to be laterally separated from each other, and a thermally melting sheet disposed between the front-surface sheet and the absorber so as to be piled up on the side sheets via the front-surface sheet. The side sheets and the thermally melting sheet are formed of synthetic fiber containing thermoplastic resin, and a fusion part is formed between the side sheets and the thermally melting sheet, so as to adhere to each other via the front-surface sheet by being thermally melted.

### Citation List

### Patent Literature

Patent Document 1 Japanese Patent Application Laid-Open Publication No. 2013-66614

### Summary of Invention

### Technical Problem

However, in the absorbent article disclosed in Patent Document 1 described above, as shown in Fig. 10, a fusion part 55 is formed, in the manner that a thermally melting sheet 52 disposed between a front-surface sheet 50 and an absorbent body 51, and a side sheet 53 arranged on the skin facing surface of the front-surface sheet 50 are mutually bonded by thermal melt via the front-surface sheet 50. Thus, although the front-surface sheet 50 is bonded between the side sheet 53 and the thermally melting sheet 52, the fusion part 55 bonding these does not reach a back-surface sheet 54. Therefore, the front-surface sheet 50 and the side sheet 53 together with the thermally melting sheet 52 may easily be lifted up when the side sheet 53 or the front-surface sheet 50 is pulled up during wearing, and thereby may be delaminated from the end part thereof and torn easily. Especially, the front and back end parts of the absorbent article with the side sheets, the front-surface sheet and the thermally melting sheet piled up are exposed to the outside, and thus are easily delaminated and torn therefrom. When the absorbent article is bent in the front and back direction along a curved body in the front and back direction, the difference in circumferential length between the inner circumference and the outer circumference of the thick absorbent article becomes large due to the thickness, the layered portion of the front-surface sheet and the back-surface sheet easily moves, and the front-surface sheet is easily lifted up.

It is therefore a major object of the present invention to provide an absorbable article including a front-surface sheet made of non-thermally fusible fiber, and preventing the front-surface sheet from delaminating and tearing accompanied therewith.

### Solution to Problem

In order to solve the above problems, as the present invention according to claim 1, an absorbable article is provided, including a front-surface sheet in a skin side and a back-surface sheet in a non-skin side with an absorber interposed therebetween, and side sheets respectively arranged on both side parts of the skin side along a longitudinal direction. The front-surface sheet contains non-thermally fusible fiber, and the back-surface sheet and the side sheets contain thermally fusible resin. Fusion parts allowing the side sheets and the back-surface sheet to be thermally fused under a state of the front-surface sheet interposed therebetween are disposed in areas corresponding to side parts outer than the absorber in a width direction, and the areas include the front-surface sheet interposed between the side sheets and the back-surface sheet, and include at least front and back end parts of the absorbable article.

In the invention of claim 1 described above, as the front-surface sheet, a sheet containing non-thermally fusible fiber such as cotton fiber is used, and as the back-surface sheet and the side sheets, a sheet containing thermally fusible resin such as synthetic fiber is used. The fusion parts allowing the side sheets and the back-surface sheet to be thermally fused under the state of the front-surface sheet interposed therebetween are disposed in the areas corresponding to the side parts outer than the absorber in the width direction, and the areas include the front-surface sheet interposed between the side sheets and the back-surface sheet, and include at least the front and back end parts of the absorbable article. In the fusion parts, although the non-thermally fusible fiber is not melted, the side sheets and the back-surface sheet containing thermally fusible resin respectively layered on the skin side and the non-skin side of the front-surface sheet are melted by heating, and entered and solidified in the gaps and the like of the fiber of the front-surface sheet, thereby allowing the side sheets, the front-surface sheet and the back-surface sheet to be bonded integrally. This enables to prevent the front-surface sheet from delaminating at least from the front and back end parts of the absorbable article, and further enables to prevent a sheet from tearing accompanied with the delamination of the front-surface sheet.

As the present invention according to claim 2, in the absorbable article of claim 1, the fusion parts are arranged individually in a front-side area and a back-side area of the absorbable article.

In the invention of claim 2 described above, the front-surface sheet is prevented from delaminating from the front and back end parts of the absorbable article where such delamination easily occurs, and the fusion parts are not arranged therebetween to avoid unpleasant feeling given to a wearer during wearing the absorbable article due to the solidified fusion parts.

As the present invention according to claim 3, in the absorbable article of claim 1, the fusion parts are arranged over a full length in the longitudinal direction of the absorbable article so as to connect a front side end edge part and a back side end edge part of the absorbable article.

In the invention of claim 3 described above, in order to more reliably prevent delamination of the front-surface sheet, the fusion parts are arranged over the full length in the longitudinal direction of the absorbable article so as to connect the front side end edge part and the back side end edge part of the absorbable article.

As the present invention according to claim 4, in the absorbable article of any one of claims 1 to 3, the fusion parts are continuously thermally fused, or the fusion parts are intermittently thermally fused.

In the invention of claim 4 described above, the fusion parts are continuously thermally fused in order to increase the strength in bonding in the fusion parts, or the fusion parts are intermittently thermally fused such as in a square lattice shape pattern or a stripe pattern with plural lines arranged in parallel, in order to suppress deterioration in wearing feeling due to the solidified fusion parts.

As the present invention according to claim 5, in the absorbable article of any one of claims 1 to 4, the side sheets are formed each in one layer; or the side sheets are folded each into two layers with a folded end part arranged toward an inside in the width direction of the absorbable article; or further alternatively the side sheets are folded each into two layers, the two-layered sheets each includes an elastic stretchable member inside, and the two-layered sheets are folded outward and bonded at the front and back end parts of the absorbable article so as to form standing gathers standing on the skin side of the absorbable article in middle portions of the longitudinal direction.

In the invention of claim 5 described above, the side sheets in embodiments are formed (1) each in one layer so as to provide simple structure, (2) each in two layers with the folded end part arranged toward the inside in the width direction of the absorbable article, so as to provide increased leakage preventing property in terms of leakage to the outside in the width direction, and (3) to have the standing gathers so as to provide further increased leakage preventing property in terms of leakage to the outside in the width direction.

As the present invention according to claim 6, in the case of the side sheets forming the standing gathers, outward folded portions of the two-layered sheets cover the skin sides of the fusion parts in the front and back end parts of the absorbable article.

In the invention of claim 6 described above, in the case of the side sheets forming the standing gathers, the outward folded portions of the two-layered sheets of the standing gathers (the portions standing on the skin side of the absorbable article in the middle portions of the longitudinal direction) cover the skin sides of the fusion parts in the front and back end parts of the absorbable article, thereby allowing to prevent the fusion parts solidified by thermo-fusion from being brought into direct contact with the skin, resulting in enabling to suppress deterioration in wearing feeling.

As the present invention according to claim 7, in the absorbable article of any one of claims 1 to 6, the front-surface sheet has many openings penetrating through the sheet from the skin side to the non-skin side, and the fusion parts are arranged in areas covering the openings.

In the invention of claim 7 described above, in the case of using the front-surface sheet having many openings penetrating through the sheet from the skin side to the non-skin side, the fusion parts are disposed in the areas covering the openings, whereby the melted thermally fusible fiber is able to be bonded mutually through the openings when the side sheets and the back-surface sheet respectively layered on the skin side and the non-skin side of the front-surface sheet are subjected to the thermo-fusion. This allows to further increase the strength in bonding.

As the present invention according to claim 8, in the absorbable article of any one of claims 1 to 7, the side sheets and the back-surface sheet contain the same kind of thermally fusible fiber.

In the invention of claim 8 described above, the side sheets and the back-surface sheet contain the same kind of thermally fusible fiber, thereby allowing the both sheets to melt at a constant temperature during thermo-fusion. This allows to further enhance the effect of the thermo-fusion.

### Advantageous Effect of Invention

As detailed above, the present invention allows the absorbable article including the front-surface sheet containing non-thermally fusible fiber to prevent the front-surface sheet from delaminating and tearing accompanied therewith.

### Brief Description of Drawings

Fig. 1 is a partially broken developed view of a sanitary napkin 1 according to the present invention.
Fig. 2 is a diagram taken along a II-II line of Fig. 1.
Fig. 3 is a diagram taken along a III-III line of Fig. 1.
Fig. 4 is an enlarged sectional view of a fusion part 10 (diagram taken along a IV-IV line).
Fig. 5 is a plan view of a sanitary napkin 1 according to a modification.
Fig. 6 are plan views of the sanitary napkin 1 with the upper right portion enlarged, illustrating planar patterns of the fusion part 10.
Fig. 7 is a cross-sectional view of a sanitary napkin 1 according to a modification (Example 1).
Fig. 8 is a cross-sectional view of a sanitary napkin 1 according to a modification (Example 2).
Fig. 9 are cross-sectional views of the sanitary napkin 1, illustrating a procedure of processing a standing gather BS.

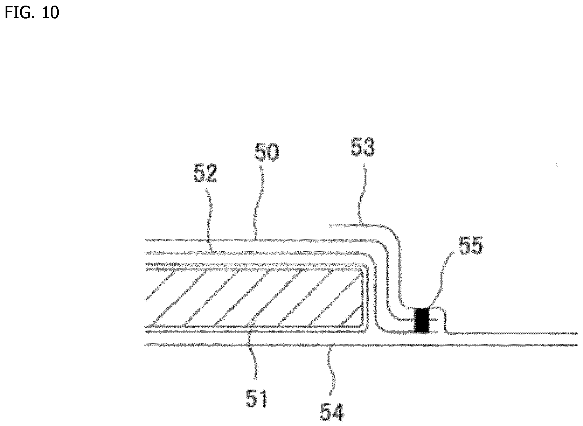

Fig. 10 is an enlarged cross-sectional view illustrating a conventional absorbable article.

### Description of Embodiments

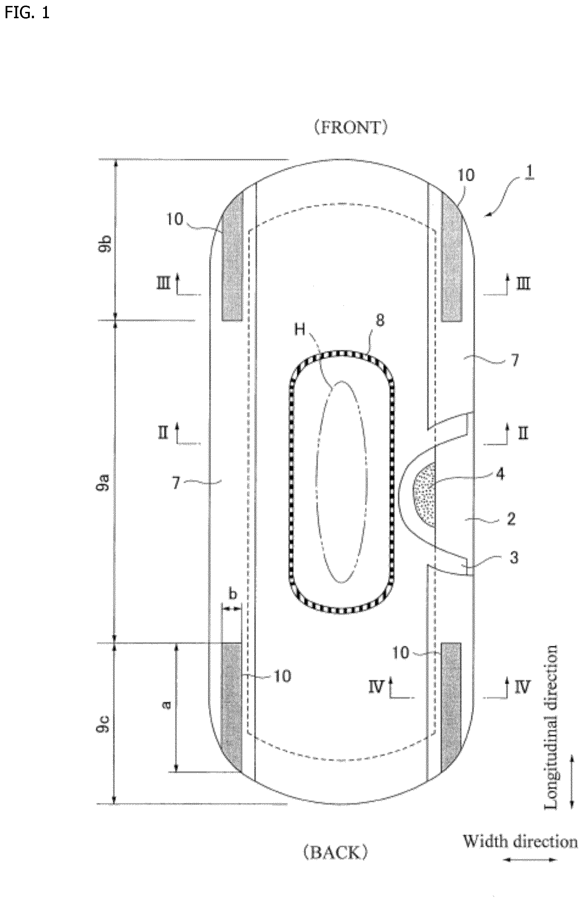

Hereinafter, some embodiments according to the present invention will be detailed with reference to the drawings. As shown in Fig. 1 to Fig. 3, a sanitary napkin 1 according to the present invention mainly includes a liquid impermeable back-surface sheet 2 formed of a polyethylene sheet or the like, a front-surface sheet 3 which serves as a skin-contact surface and promptly transmits body fluid, an absorber 4 which is interposed between the both sheets 2, 3 and is made of cotton-like pulp, synthetic pulp or the like, and side sheets 7 which are arranged respectively on the both side parts of the skin side of the front-surface sheet 3 along the longitudinal direction thereof. Around the absorber 4, the outside edge parts of the back-surface sheet 2 and the front-surface sheet 3 may be bonded by a hot melt adhesive or the like at least in a part of the upper and lower end edge parts of the absorber 4. Alternatively, the parts extending laterally over the absorber 4 of the back-surface sheet 2 and the side sheets 7 may be bonded by bonding means such as a holt melt adhesive or heat sealing at least in a part of the both side edge parts of the absorber 4. It is noted that none of these types of bonding means may be performed in a section where a fusion part 10 to be described later is arranged.

### Back-Surface Sheet

As the back-surface sheet 2, a sheet material having at least water impermeability such as polyethylene is used. Recently, material having moisture permeability tends to be used from the viewpoint of stuffiness prevention. As such a water impermeable and moisture permeable sheet material, a microporous sheet is preferably used, which is obtained by melt-kneading inorganic filler in olefin resin such as polyethylene or polypropylene and forming a sheet, and thereafter extending the sheet in one axial direction or two axial directions.

The back-surface sheet 2 is formed of thermally fusible fiber. As the thermally fusible fiber, any type of fiber is available, as long as it is melted by heating and expresses mutual bonding property. The thermally fusible fiber may be made of a single type of fiber, or may be composite fiber or the like obtained by combining two types or more of synthetic resin. Specifically, such fiber may be a single polyolefin fiber such as polyethylene, polypropylene or polyvinyl alcohol, sheath/core composite fiber or eccentric sheath/core composite fiber with a sheath part having a relatively low melting point, made of polyethylene terephthalate/polyethylene, polyethylene terephthalate/polypropylene, polypropylene/polyethylene, polyethylene-terephthalate-ethylene-propylene copolymer or low-melting polyester-polyester, split-type composite fiber with a component partially exposed to a surface, made of polyethylene terephthalate/polypropylene, polyethylene terephthalate/nylon or polo propylene/polyethylene, or thermally-spit-type composite fiber split by thermal shrinking of one component, made of polyethylene terephthalate/ethylene-propylene copolymer. In the case of focusing on productivity and dimensional stability, sheath/core composite fiber is preferably used. In the case of focusing on voluminous feeling of nonwoven fabric, eccentric sheath/core composite fiber is preferably used. In the case of focusing on softness, split-type composite fiber or thermally-split-type composite fiber is used, in which each component is easily split and made into ultrafine fiber during high-pressure water stream treatment.

### Front-Surface Sheet

The front-surface sheet 3 forms a skin-contact surface covering the skin side of the absorber 4, and is formed of non-thermally fusible fiber. As the non-thermally fusible fiber, any type of fiber is available, as long as it is not melted by heating or expresses no bonding property between the non-thermally fusible fiber. Examples of the non-thermally fusible fiber are natural fiber such as cotton, pulp, silk and lyocell, regenerated cellulose fiber such as rayon and cupra, and semi-synthetic fiber such as acetate. Among them, natural fiber is preferably used owing to its good touch. Particularly, cotton fiber or lyocell fiber, among the natural fiber, may be used owing to its excellent touch and its excellent water absorbency and hygroscopicity. The front-surface sheet 3 may be formed of spun lace nonwoven fabric with 100 wt. % of cotton fiber, alternatively may be formed of laminated spun lace nonwoven fabric with 100 wt. % of cotton fiber as the skin side layer and with thermally fusible fiber as the non-skin side layer. The spun lace nonwoven fabric has advantages such as softness and none-use of adhesive.

As the cotton fiber, any type of cotton is available, such as raw cotton, scoured and bleached cotton fiber or dyed cotton fiber after subjected to scouring and bleaching, scoured and bleached degreased cotton, and recovered wool obtained by defibrating yarn or fabric. Especially, non-degreased cotton is preferably used, having slight water-repellant property even under the state of fiber owing to the natural fat and oil of cotton wax attached to the surface of the cotton fiber.

The front-surface sheet 3 has a fabric weight per unit area in a range from 20 g/m² to 40 g/m², preferably in a range from 27 g/m² to 34 g/m², more preferably in a range from 29 g/m² to 32 g/m², and has a thickness in a range from 0.25 mm to 0.50 mm, preferably in a range from 0.3 mm to 0.4 mm. The fabric weight per unit area is obtained by measuring the weight of 10 sheets each having the size of 5cm x 4cm with an electronic balance and calculating weight per square meter. The thickness is able to be measured by Digital Type FFD-7 of Constant Pressure Thickness Gauge manufactured by OZAKI MFG. CO., LTD.

The front-surface sheet 3 may be formed to have many openings penetrating through the sheet from the skin side to the non-skin side in the thickness direction, in order to enhance the fluid permeability. Specifically, the openings may be formed by making a mesh-like support body support fiber material in a hydroentanglement step during spun lace production. In this case, the change of the conditions of the mesh to be used allows to adjust the individual opening size and the opening ratio. The openings may be simply formed in the manner that the nonwoven fabric after production is subjected to punching. The openings are formed in at least the area where the fusion part 10 to be described later is provided, preferably additionally formed in a body fluid expelling portion H and its vicinity in order to provide higher water permeability in the body fluid expelling portion H, further preferably formed on the entire surface sheet.

In the case where the front-surface sheet 3 is formed of the spun lace nonwoven fabric made of 100 wt. % of cotton fiber coated with water-repellant agent and is formed to have many openings penetrating through the sheet from the skin side to the non-skin side, the front-surface sheet 3 has advantages of providing soft touch and hardly causing skin trouble during wearing such as itching and rash even after wearing for a long time, and further suppressing a wearer from feeling stuffiness before expelling of body fluid owing to the hygroscopicity of the cotton fiber. In this case, the problem in terms of the residue of the fluid on the surface is sufficiently solved by the coated water-repellant agent. In the case of the front-surface sheet 3 having many openings in the area including the body fluid expelling portion H, the front-surface sheet 3 is capable of promptly transmitting body fluid through the openings.

### Absorber 4

The absorber 4 interposed between the back-surface sheet 2 and the front-surface sheet 3 includes, for example, cotton-like pulp and a water absorbent polymer. The water absorbent polymer, for example, as granular powder, is mixed in the pulp included in the absorber. The pulp is made of cellulose fiber such as chemical pulp or melted pulp obtained from wood, or artificial cellulose fiber such as rayon or acetate. Needle leaved tree pulp having a longer fiber length than that of broad-leaved tree pulp is preferably used from the viewpoint of function and cost. The absorber 4 has a fabric weight per unit area in a range from 290 g/m² to 1000 g/m², preferably in a range from 390 g/m² to 850 g/m², more preferably in a range from 630 g/m² to 720 g/m².

The absorber 4 may include mixed synthetic fiber. Specifically, the synthetic fiber may be polyolefin system such as polyethylene or polypropylene, polyester system such as polyethylene terephthalate or polybutylene terephthalate, polyamide system such as nylon, or a copolymer thereof. Two kinds among them may be mixed and used. Furthermore, composite fiber is available, such as sheath/core fiber with high-melting point fiber as a core and low-melting point fiber as a sheath, side-by-side fiber, and split-type fiber. In the case where the synthetic fiber is hydrophobic fiber, the synthetic fiber is desirably used after subjected to surface treatment with a hydrophilizing agent, so as to impart affinity to body fluid.

As the absorber 4, a polymer sheet with granular powder superabsorbent polymer supported between two upper and lower layers of hydrophilic sheets made of unwoven fiber, paper or the like may be used. The polymer sheet has only superabsorbent polymer supported between two layers of sheets, without pulp fiber. Between the pulp sheet and the front-surface sheet 3, an intermediate sheet made of hydrophilic nonwoven fabric may be arranged, when needed.

Furthermore, the absorber 4 may be formed by pilling up an absorber including pulp fiber and superabsorbent polymer on the skin side or the non-skin side of the polymer sheet. As for the combination of the polymer sheet and the absorber including the pulp fiber and the superabsorbent polymer, the absorber may be formed by pilling up one layer for each, or may be formed by making plural layers of either one of them or both of them, and pilling up them alternately.

Examples of the superabsorbent polymer are cross-linking polyacrylate, self-cross-linking polyacrylate, saponified substance of cross-linking copolymer of acrylic acid ester and vinyl acetate, cross-linking substance of copolymer of isobutylene and maleic anhydride, cross-linking polysulfonate, and partially cross-linking substance of water swelling polymer such as polyethylene oxide and polyacrylamide. Among them, substance of acryl acid or acrylate-based substance excellent in amount of water absorption and water absorption rate is preferably used. The absorption ratio (water absorbing power) and the absorption rate of the superabsorbent polymer having the above-described water absorbing property are adjustable, by adjusting the cross-linking density and the cross-linking density gradient in its manufacturing process.

In the case of the absorber 4 containing the pulp fiber, the absorber 4 is desirably wrapped by an encapsulating sheet 5 formed of a crepe paper sheet, nonwoven fabric or the like, in order to retain the shape and support polymer powder, and other reasons.

The absorber 4 has, on the skin side surface thereof, an emboss groove 8 forming an appropriate planar shape where the front-surface sheet 3 and the absorber 4 are integrally dented to the non-skin side, so as to cover at least the area corresponding to the body fluid expelling portion H of a wearer at least in the width direction. The formation of the emboss groove 8 allows the absorber 4 to reliably absorb the body fluid flowing into the emboss groove 8. The planar shape of the emboss groove 8 may be in, for example, a substantially oval shape as shown in Fig. 1, or a substantially elliptical shape, or may be any other known shape as long as it is arranged so as to cover at least the area corresponding to the body fluid expelling portion H of a wearer at least in the width direction.

As shown in Fig. 1, the sanitary napkin 1 is sectioned in the longitudinal direction into a central area 9a which is the section corresponding to the body fluid expelling portion H of a wearer, that is, two central sections out of the four equally-divided sections of the sanitary napkin 1 in the longitudinal direction thereof, a front side area 9b which is the section positioned in the front side of the central area 9a, and a back side area 9c which is the section positioned in the back side.

### Side Sheet

As in the illustrated example, the front-surface sheet 3 is formed wider by a predetermined width than the width of the absorber 4. The outside parts of the front-surface sheet 3 in the width direction are covered by the side sheets 7 (different members from the front-surface sheet 3) respectively extending from the both side parts of the skin side surface of the front-surface sheet 3. The side sheets 7 are arranged respectively on the both side parts separated by the center in the width direction.

Each of the side sheets 7, as with the back-surface sheet 2, is made of thermally fusible fiber. As the thermally fusible fiber, any type of fiber is available, as long as it is melted by heating and expresses mutual bonding property. As with the back-surface sheet 2, the thermally fusible fiber may be made of a single type of fiber, or may be composite fiber or the like obtained by combining two types or more of synthetic resin. Specifically, such fiber may be single polyolefin fiber such as polyethylene, polypropylene or polyvinyl alcohol, sheath/core composite fiber or eccentric sheath/core composite fiber with a sheath part having a relatively low melting point, made of polyethylene terephthalate/polyethylene, polyethylene terephthalate/polypropylene, polypropylene/polyethylene, polyethylene-terephthalate-ethylene-propylene copolymer or low-melting polyester-polyester, split-type composite fiber with a component partially exposed to a surface, made of polyethylene terephthalate/polypropylene, polyethylene terephthalate/nylon or polo propylene/polyethylene, or thermally-spit-type composite fiber split by thermal shrinking of one component, made of polyethylene terephthalate/ethylene-propylene copolymer. In the case of focusing on productivity and dimensional stability, sheath/core composite fiber is preferably used. In the case of focusing on voluminous feeling of nonwoven fabric, eccentric sheath/core composite fiber is preferably used. In the case of focusing on softness, split-type composite fiber or thermally-split-type composite fiber is used, in which each component is easily split and made into ultrafine fiber during high-pressure water stream treatment.

As the side sheet 7, nonwoven fabric material appropriately subjected to appropriate water-repellant treatment or hydrophilic treatment is available depending on the purpose such as of preventing body fluid and the like from permeating or enhancing the texture.

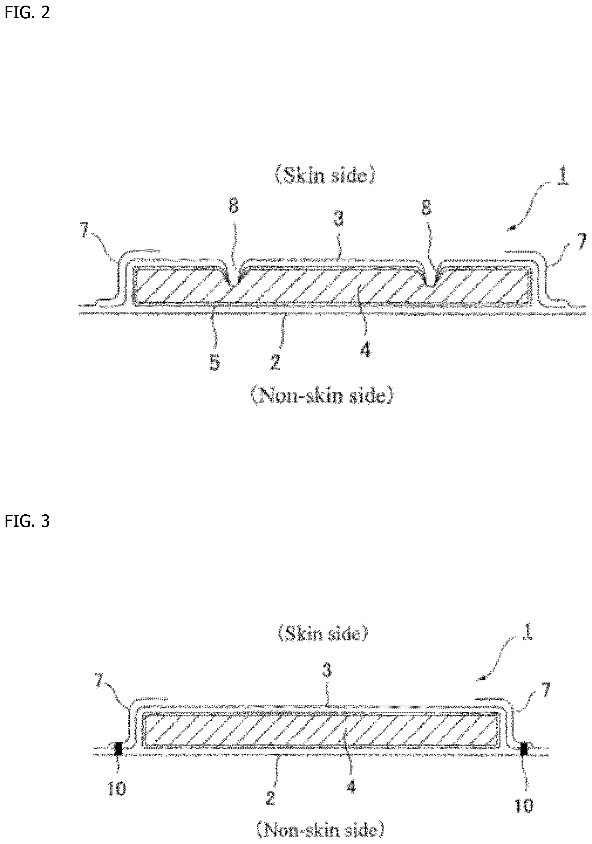

As shown in Fig. 2 and Fig. 3, the side sheets 7 are arranged in the ranges outside the central portion in the width direction from the inside of the absorber 4 slightly beyond the side edge parts of the absorber to the outer edge parts of the back-surface sheet 2, and appropriate areas are bonded by a hot melt adhesive or the like. The side sheets 7 shall not be bonded by an adhesive on the portion where the fusion part 10 to be described later is provided, in order to prevent the side sheets 7 and the back-surface sheet 2 from being lowered in the fusion by thermal melt, but may be bonded in the area where such fusion is not hindered.

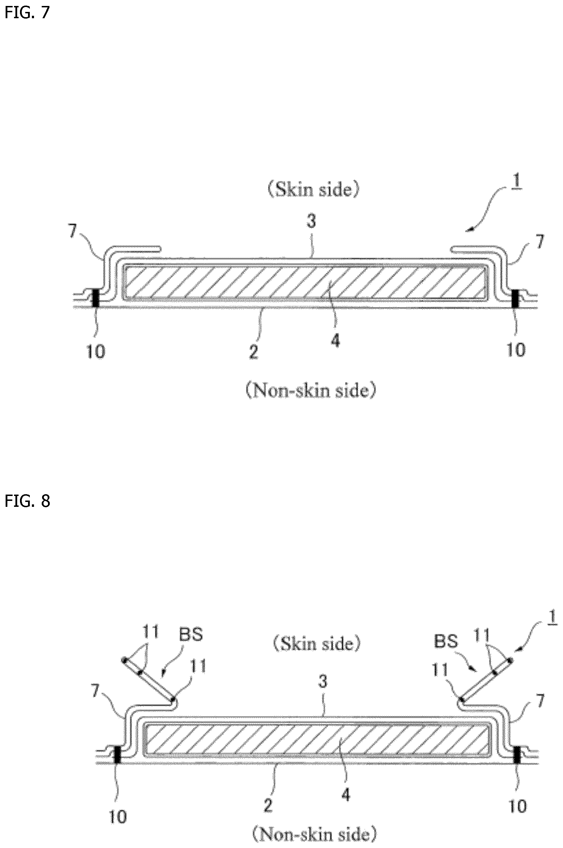

The side sheets 7 are formed each in one layer in the example shown in Fig. 1 to Fig. 3, or may be folded each in, as in the following description, two layers in which the folded end thereof is arranged toward the inside in the width direction of the sanitary napkin 1 (refer to Fig. 7). Alternatively, the folded two-layered sheets may include elastic stretchable members 11 inside, and the two-layered sheets may be folded outward and bonded at the front and back end parts of the sanitary napkin 1, thereby forming standing gathers BS standing on the skin side of the sanitary napkin 1 in the middle portions of the longitudinal direction (refer to Fig. 8).

### Fusion Part

As shown in Fig. 1, Fig. 3 and Fig. 4, thermo-fusion parts 10 thermally fused under the state where the front-surface sheet 3 is interposed between the side sheets 7 and the back-surface sheet 2 are arranged in the areas which are the side parts outer than the absorber 4 in the width direction, have the front-surface sheet 3 interposed between the side sheets 7 and the back-surface sheet 2, and include at least the front and back edge parts of the sanitary napkin 1. In the thermo-fusion parts 10, although the non-thermally fusible fiber of the front-surface sheet 3 is not melted, the side sheets 7 and the back-surface sheet 2 containing thermally fusible resin respectively layered on the skin side and the non-skin side of the front-surface sheet 3 are melted by heating, and the melted fiber is entered and solidified in the gaps of the fiber of the front-surface sheet 3 and the many openings formed in the front-surface sheet 3. This allows the side sheets 7, the front-surface sheet 3 and the back-surface sheet 2 to be bonded integrally. Especially, in the case of the front-surface sheet 3 having many openings, the front-surface sheet 3 allows the melted thermally fusible fiber of the side sheets 7 and the back-surface sheet 2 to be bonded to each other through the openings, thereby achieving stronger bonding. The bonding by the fusion parts 10 corresponds to structural bonding by melting and solidifying the thermally fusible fiber included in the side sheets 7 and the back-surface sheet 2, and thus achieves stronger bonding than the adhesion between two layers by application of an adhesive, thereby enabling to reliably prevent their delamination.

The thermo-fusion parts 10 are formed on the flap parts where the side sheets 7, the front-surface sheet 3 and the back-surface sheet 2 are adjacently layered in this order from the skin side, without the absorber 4 interposed, in the areas which are the side parts outer than the absorber 4 in the width direction and do not cover the absorber 4 in the thickness direction.

The fusion parts 10 are formed in the areas at least including the front and back end parts of the sanitary napkin 1. The description of "including the front and back end parts of the sanitary napkin 1" means being formed to cover the end edge parts of the sanitary napkin 1 in the longitudinal direction (front-and-back direction). This allows the end edge parts of the front-surface sheet 3 arranged in the front and back end edge parts of the sanitary napkin 1 to be firmly fixed to the side sheets 7 and the back-surface sheet 2, thereby enabling to reliably prevent delamination from the edge parts.

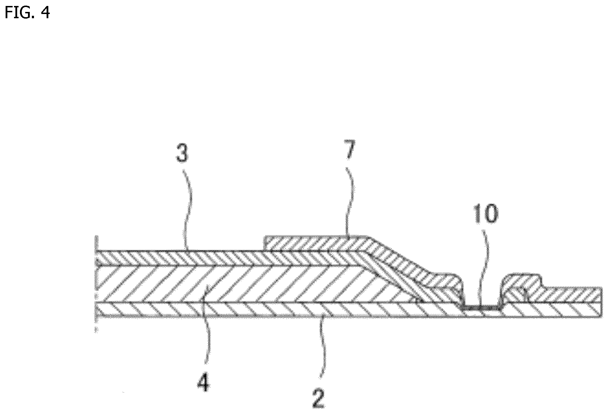

In forming each of the fusion parts 10, as shown in Fig. 4, the side sheet 7, the front-surface sheet 3 and the back-surface sheet 2 are made to pass between the embossing roll having an embossed projecting part formed on the surface thereof and the anvil roll having a flat surface, and compressed from the skin side of the side sheet 7 and concurrently heated to the melting point or above of the thermally fusible fiber included in the side sheet 7 and the back-surface sheet 2, whereby the thermally fusible fiber is melted. At the fusion part 10, the side sheet 7, the front-surface sheet 3 and the back-surface sheet 2 are compressed to the non-skin side, so that the concave part recessed to the non-skin side of the side sheet 7 is formed.

As shown in Fig. 1, the fusion parts 10 are disposed respectively in the front side area 9b and the back side area 9c of the sanitary napkin 1, not in the central area 9a. This allows to prevent delamination in the front and back end parts of the sanitary napkin 1 in which the front-surface sheet 3 easily delaminates, and further allows to suppress unpleasant feeling and skin trouble during wearing the sanitary napkin 1 in the central area 9a, due to the contact of the skin with the fusion parts 10 solidified by thermal melt.

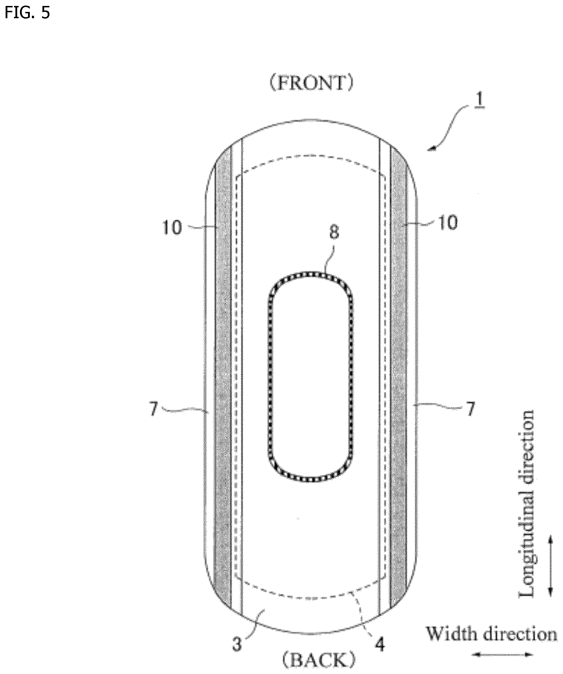

Alternatively, as shown in Fig. 5, the fusion parts 10 may be arranged respectively over the full length of the sanitary napkin 1 in the longitudinal direction so as to connect the front side end edge parts and the back side end edge parts of the sanitary napkin 1. This allows to increase the strength in bonding by the fusion parts 10, and allows to prevent delamination of the front-surface sheet 3 over the full length of the sanitary napkin 1. The thermo-fused portions are formed over the full length of the sanitary napkin 1 on the both side parts of the absorber 4, thereby enabling to reliably prevent leakage from the side edge parts of the sanitary napkin 1.

The dimensions of the fusion parts 10 are described on the basis of Fig. 1. A length a in the longitudinal direction of the sanitary napkin 1 is preferably set to 8mm or more, respectively from the front and back end edge parts of the sanitary napkin 1. If the length a is shorter than 8mm, the fusion parts 10 have low strength in bonding, and thus delamination occurs easily. In the case where the fusion parts 10 are arranged individually in the front side area 9b and the back side area 9c, the fusion parts 10 shall have the length satisfying that the end parts thereof in the inner sides of the longitudinal direction of the sanitary napkin 1 do not reach the central area 9a. As described above, the fusion parts 10 may be disposed over the full length of the sanitary napkin 1.

A width b of the fusion parts 10 is preferably set to a range from 2 mm to 15 mm. If the width b is shorter than 2 mm, the fusion parts 10 have low strength in bonding, and thus delamination occurs easily. If the width b is longer than 15mm, the fusion parts 10 are formed too hard and thus the wearing feeling is deteriorated. In order to ensure the width b of the fusion parts 10, the side sheets 7, the front-surface sheet 3 and the back-surface sheet 2 are respectively extended by predetermined lengths to the outer sides in the width direction from the absorber 4. The fusion parts 10 are disposed on the center parts in the width direction of the flap parts disposed outside the absorber 4 in the width direction, so as not to reach the side edge parts of the sanitary napkin 1. That is, outside the fusion parts 10 in the width direction, the flap parts are formed, in which at least the side sheets 7 and the back-surface sheet 2 are layered but the fusion parts 10 are not formed. Therefore, the side edge parts of the sanitary napkin 1 provides a soft feeling to the skin during wearing the sanitary napkin 1, thereby suppressing deterioration in wearing feeling.

As shown in Fig. 1, the fusion parts 10 are able to be formed by continuously compressing and thermo-fusing predetermined areas over the entire surface. This increases the strength in bonding of the fusion parts 10, thereby enabling to reliably prevent the front-surface sheet 3 from delaminating.

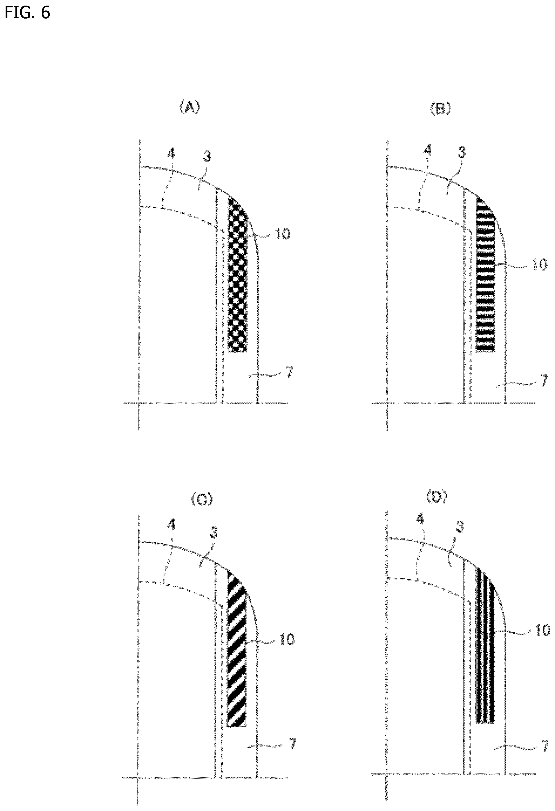

As shown in Fig. 6, each of the fusion parts 10 may be formed by intermittently compressing and thermo-fusing predetermined areas. In Fig. 6, the portions in black have been compressed and thermo-fused, and the other portions are intermittent portions not having been compressed or thermo-fused. Specifically, Fig. 6 (A) shows the formation in a square lattice shape pattern; Fig. 6 (B) shows a stripe pattern along the width direction; Fig. 6 (C) shows a stripe pattern along the oblique direction; and Fig. 6 (D) shows a stripe pattern along the longitudinal direction. Such intermittent formation of the fusion part 10 allows to suppress the deterioration in wearing feeling due to the solidified fusion part 10. In the case of intermittent formation, in order to ensure the strength in bonding, the area subjected to compressing and thermo-fusing is preferably 50% or more of the entire area of the fusion part 10, more preferably in a range from 50% to 70%.

As shown in Fig. 3, the side sheets 7 are available each in one layer of the state where one sheet of the side sheet 7 is arranged as it is. In this case, although the structure thereof is simple and thus the manufacturing cost is low, the structure hardly exhibits sufficient effect of preventing side leakage.

Alternatively, as shown in Fig. 7, the side sheets 7 may be folded each in two layers in which the folded end thereof is arranged toward the inside in the width direction of the sanitary napkin 1. This allows to increase leakage preventing property in terms of leakage to the outside in the width direction.

As shown in Fig. 8, the side sheets 7 each includes, inside the folded two-layered sheet structure, one or a plural of (three in the illustrative example) threadlike elastic stretchable members 11,11 which is disposed in the middle portion in the height direction and fixed at the both end parts or at an appropriate location in the longitudinal direction. The two-layered sheet parts folded once outward and piled at the front and back end parts thereof may be bonded to the side of the back-surface sheet 2, thereby forming a pair of the right and left linear standing gathers BS, BS standing on the skin side while inclining outward. This allows to further increase the leakage preventing property in terms of leakage to the outside in the width direction.

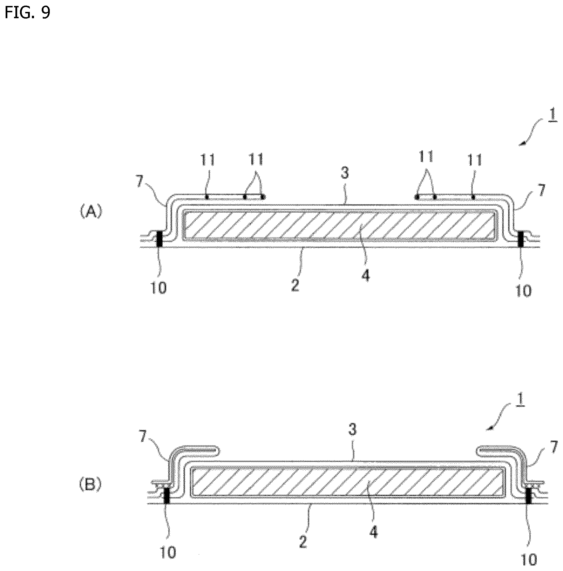

To form the standing gathers BS shown in Fig. 8, as shown in Fig. 9 (A), first, the fusion parts 10 are formed at predetermined positions under the state where the two-layered sheets are extended inward in the width direction, and thereafter as shown in Fig. 9 (B), the two-layered parts are folded once outward and bonded to the side of the back-surface sheet 2 at the front and back end parts of the sanitary napkin 1. This forms the standing gathers BS with the middle portions thereof in the longitudinal direction standing toward the skin, as shown in Fig. 8.

At this time, as shown in Fig. 9 (B), the two-layered sheet parts are preferably folded once outward so as to cover the skin sides of the fusion parts 10 in the front and back end parts of the sanitary napkin 1. This allows to prevent the fusion parts 10 solidified by thermal melt from being brought into direct contact with the skin, thereby enabling to suppress deterioration in wearing feeling.

The side sheets 7 and the back-surface sheet 2 shall contain the same kind of thermally fusible fiber. The containing of the same kind of thermally fusible fiber allows the both sheets to be thermally melted at a constant temperature during thermo-fusing, and facilitates mutual bonding because the thermally melted fiber of the side sheets 7 and the back-surface sheet 2 permeates the front-surface sheet 3, resulting in further enhancing the effect of thermo-fusing.

### Reference Signs List

1: SANITARY NAPKIN, 2: BACK-SURFACE SHEET, 3: FRONT-SURFACE SHEET, 4: ABSORBER, 5: ENCAPSULATING SHEET, 7: SIDE SHEET, 8: EMBOSS GROOVE, 9a: CENTRAL AREA, 9b: FRONT SIDE AREA, 9c: BACK SIDE AREA, 10: FUSION PART, 11: THREADLIKE ELASTIC STRETCHABLE MEMBER

## Claims

1. An absorbable article comprising:
a front-surface sheet in a skin side and a back-surface sheet in a non-skin side with an absorber interposed therebetween; and
side sheets respectively arranged on both side parts of the skin side along a longitudinal direction, wherein
the front-surface sheet contains non-thermally fusible fiber, and the back-surface sheet and the side sheets contain thermally fusible resin, and wherein
fusion parts allowing the side sheets and the back-surface sheet to be thermally fused under a state of the front-surface sheet interposed therebetween are arranged in areas corresponding to side parts outer than the absorber in a width direction, and the areas include the front-surface sheet interposed between the side sheets and the back-surface sheet, and include at least front and back end parts of the absorbable article.

2. The absorbable article according to claim 1, wherein
the fusion parts are arranged individually in a front-side area and a back-side area of the absorbable article.

3. The absorbable article according to claim 1, wherein
the fusion parts are arranged over a full length in the longitudinal direction of the absorbable article so as to connect between a front side end edge part and a back side end edge part of the absorbable article.

4. The absorbable article according to any one of claims 1 to 3, wherein
the fusion parts are continuously thermally fused, or the fusion parts are intermittently thermally fused.

5. The absorbable article according to any one of claims 1 to 4, wherein
the side sheets are formed each in one layer,
the side sheets are folded each into two layers with a folded end part arranged toward an inside in the width direction of the absorbable article, or
the folded two-layered sheets include elastic stretchable members inside, and the two-layered sheets are folded outward and bonded at the front and back end parts of the absorbable article so as to form standing gathers standing on the skin side of the absorbable article in middle portions of the longitudinal direction.

6. The absorbable article according to claim 5, wherein
when the side sheets form the standing gathers, outward folded portions of the two-layered sheets cover the skin sides of the fusion parts in the front and back end parts of the absorbable article.

7. The absorbable article according to any one of claims 1 to 6, wherein
the front-surface sheet has many openings penetrating through the sheet from the skin side to the non-skin side, and the fusion parts are disposed in areas covering the openings.

8. The absorbable article according to any one of claims 1 to 7, wherein
the side sheets and the back-surface sheet contain a same kind of thermally fusible fiber.
